# EUROPÄISCHE PATENTSCHRIFT

(11) **EP 1 833 814 B1**
(45) Veröffentlichungstag und Bekanntmachung des Hinweises auf die Patenterteilung: **27.03.2013**
(21) Anmeldenummer: 05819258.4
(22) Anmeldetag: 14.12.2005
(51) Int. Cl.: C07D 333/38

(54) **VERFAHREN ZUR HERSTELLUNG VON SUBSTITUIERTEN 2-ALKOXYCARBONYL-3-AMINOTHIOPHENEN**
METHOD FOR THE PRODUCTION OF SUBSTITUTED 2-ALKOXYCARBONYL-3-AMINOTHIOPHENES
PROCEDE POUR PRODUIRE DES 2-ALCOXYCARBONYLE-3-AMINOTHIOPHENES SUBSTITUES

(30) Priorität: 29.12.2004 DE 102004063191
(43) Veröffentlichungstag der Anmeldung: 19.09.2007
(73) Patentinhaber: Bayer CropScience Aktiengesellschaft, 40789 Monheim (DE)
(72) Erfinder: GELLER, Thomas, 51519 Odenthal (DE)
(86) Internationale Anmeldenummer: PCT/EP2005/013406
(87) Internationale Veröffentlichungsnummer: WO 2006/072375

(56) Entgegenhaltungen:
- EP-A- 0 331 919
- WO-A-03/062221
- CONFALONE P. N. ET AL.: J. ORG. CHEM., Bd. 42, Nr. 9, 1977, Seiten 1630-1633, XP002388421
- ROSSY, PHILLIP A.; HOFFMANN, WERNER; MUELLER, NORBERT: JOURNAL OF ORGANIC CHEMISTRY, vol. 45, no. 4, 1980, page 617-620,

## Beschreibung

Die Erfindung betrifft ein neues Verfahren zur Herstellung von substituierten 4-Alkoxycarbonyl-3-aminothiophenen, welche als Zwischenprodukte für Wirkstoffe in der Landwirtschaft, insbesondere für substituierte, herbizid aktive Thienylaminocarbonyltriazolinone (vgl. WO 01/05788), bekannt sind.

Es ist bekannt, dass substituierte 4-Alkoxycarbonyl-3-aminothiophene durch Oxidation mit Wasserstoffperoxid und anschließende saure Aufarbeitung hergestellt werden können (EP-A 331 919). Bei diesen Verfahren sind die erzielbaren Ausbeuten jedoch nicht völlig zufrieden stellend. Des Weiteren wurde beschrieben, dass 4-Alkoxycarbonyl-3-aminothiophene durch Umsetzung von 3-Oxatetrahydrothiophenen mit einem Säureadditionssalz von Hydroxylamin umgesetzt und die erhaltenen Oxime mit einer Säure behandelt (DE-A 27 37 738) bzw. in situ zu den entsprechenden Aminhydrochloriden umgesetzt werden können. Nachteilig bei dieser Reaktion ist jedoch das beobachtete Auftreten von decarboxyliertem Amin als unerwünschtem Beiprodukt, eine aufwendige Aufreinigung und die Notwendigkeit, das Hydroxylamin-Säureadditionssalz in großem Überschuss einzusetzen.

Die Herstellung der substituierten 4-Alkoxycarbonyl-3-aminothiophene auf einem anderen Weg bei höherer Ausbeute und Effizienz ist daher wünschenswert.

Erfmdungsgemäß wurde nun gefunden, dass die Herstellung der substituierten 4-Alkoxycarbonyl-3-aminothiophene in hoher Ausbeute gelingt, wenn man 3-Oxatetrahydrothiophenen mit Ammoniumacetat oder Ammoniumformiat zu den entsprechenden Enaminen umsetzt. Diese Umsetzung gelingt überraschenderweise auch mit katalytischen Mengen der Salze, wenn man zusätzlich Ammoniak zusetzt. Nur mit Ammoniak und ohne den Salzzusatz findet keine nennenswerte Umsetzung zu den Enaminen statt. Die Enamine werden dann mit Chlorierungsmitteln wie Sulfurylchlorid oder Chlor umgesetzt. Überraschend verläuft die Produktbildung ohne nennenswerte Bildung von Nebenprodukten, obwohl literaturbekannt ist, dass Tetrahydrothiophene z.B. mit Sulfurylchlorid unter S-Chlorierung reagieren und Chlorkomplexe (z.B. Sulfoniumsalze) bilden (JACS, 1973, 95, 6508-6509). Diese Verbindungen sind hochreaktiv und können z.B. mit aromatischen Aminen reagieren (JACS, 1973, 95, 6508-6509) oder polymerisieren (J. Org. Chem., 1985, 50, 2840 - 2847).

Es wurde demnach gefunden, dass man 4-Alkoxycarbonyl-3-aminothiophene der allgemeinen Formel (I) 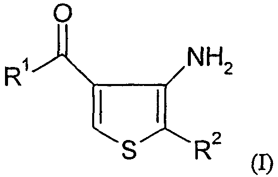 und/oder deren Hydrochloride der Formel (I)' 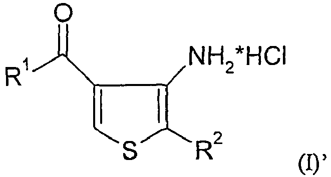 und/oder deren mono- oder bis-acetylierte bzw. mono- oder bis-formylierte Form der Formeln (I)" 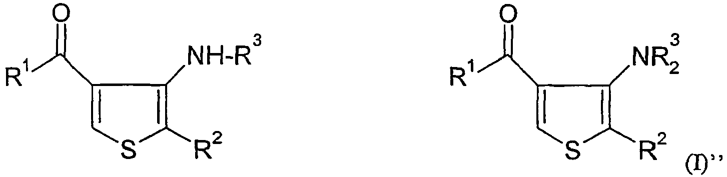 in welchen
- R¹: für gegebenenfalls durch Halogen oder C₁-C₄-Alkoxy substituiertes Alkoxy mit 1 bis 6 Kohlenstoffatomen steht,
- R²: für gegebenenfalls durch Halogen oder C₁-C₄-Alkoxy substituiertes Alkyl mit 1 bis 6 Kohlenstoffatomen oder für jeweils gegebenenfalls durch Halogen, C₁-C₄-Alkyl oder C₁-C₄-Alkoxy substituiertes Aryl oder Arylalkyl mit jeweils 6 oder 10 Kohlenstoffatomen in der Arylgruppe und gegebenenfalls 1 bis 4 Kohlenstoffatomen im Alkylteil steht, und
- R³: für Acyl- oder Formyl steht,

in sehr guten Ausbeuten und in hoher Reinheit erhält, wenn man mit Enamine der Formel (II) 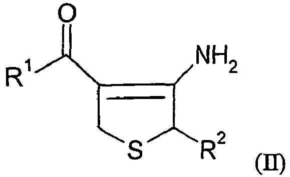 und/oder deren mono- oder bis-acetylierte bzw. mono- oder bis-formylierte Form der Formel (II)" 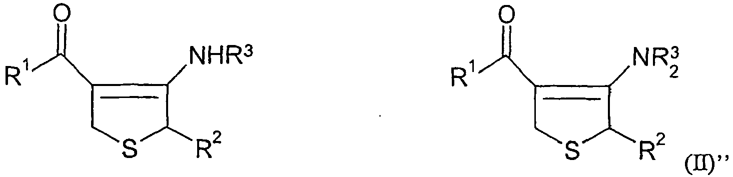 in welchen R²,R² und R³ jeweils die oben angegebene Bedeutung haben,
mit einem Chlorierungsmittel in Gegenwart eines oder mehrerer Verdünnungsmittel ohne Hilfsbasenzusatz bei Temperaturen zwischen -10°C und 0°C umsetzt.

In der Formel (I) steht R¹ bevorzugt für C₁-C₄-Alkoxy, insbesondere Methoxy, Ethoxy, n-oder i-Propoxy. R² steht bevorzugt für C₁-C₄-Alkyl, insbesondere Methyl, Ethyl, n- oder i-Propyl.

Das erfindungsgemäße Verfahren eignet sich besonders vorteilhaft zur Herstellung von 2-Methyl-3-amino-4-methoxycarbonyl-thiophen der Formel (Ia) 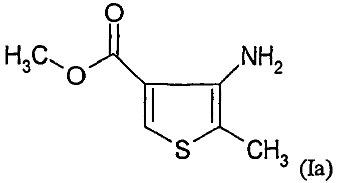 bzw. dessen Hydrochlorids der Formel (Ia)' 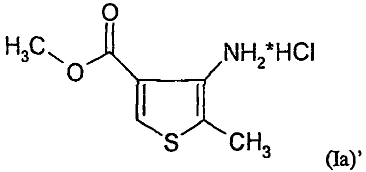 bzw. seiner mono- oder bis-acetylierten oder mono- oder bis-formylierten Form (Ia)"

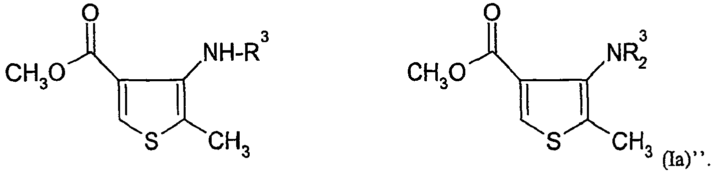

Das erfindungsgemäße Verfahren wird ohne Hilfsbasenzusatz durchgeführt.

Durch die Umsetzung der Enamine mit einem Chlorierungsmittel, wurde überraschend ein neues Verfahren gefunden, welches die Herstellung der 4-Alkoxycarbonyl-3-aminothiophene in sehr guten Ausbeuten und in hoher Reinheit bei kurzer Reaktionszeit erlaubt.

In einer bevorzugten Ausführungsform wird das Reaktionsprodukt schnell und mit hoher Ausbeute gebildet. Nach der der Umsetzung kann das Produkt durch Ausfällen als Hydrochlorid aus einer organischen Lösung mit Wasser und anschließende Filtration sehr einfach isoliert werden. Alternativ kann eine Produktisolierung im Falle der Verwendung von Sulfurylchlorid oder Chlorgas auch durch einfaches Entfernen des Lösungsmittels und des überschüssigen Oxidationsmittels unter vermindertem Druck erfolgen. Hohe Überschüsse an teuren Reagenzien sind für die Produktherstellung nicht notwendig.

Das erfindungsgemäße Verfahren stellt somit eine Bereicherung des Standes der Technik dar, da es eine sehr vorteilhafte Herstellung von substituierten 4-Alkoxycarbonyl-3-aminothiophenen erlaubt. Damit wird der Zugang zu den auf diesen Zwischenprodukten basierenden herbiziden Thienylaminocarbonyltriazolinonen erleichtert.

Die beim erfindungsgemäßen Verfahren als Ausgangsverbindungen zu verwendenden Enamine der Formel (II) werden bevorzugt durch Umsetzung von Verbindungen der Formel (III) 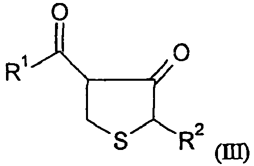 worin R¹ und R² die oben angegebene Bedeutung haben,
mit Ammoniumformiat gegebenenfalls in Gegenwart von NH₃, mit Ammoniumacetat gegebenenfalls in Gegenwart von NH₃, mit einem Gemisch aus Ameisensäure und NH₃ und/oder mit einem Gemisch aus Essigsäuresäure und NH₃ in Gegenwart eines oder mehrerer Verdünnungsmittel erhalten.

Die Verbindungen der Formel (II) können jedoch auch so hergestellt werden, wie es in der EP-A 331 919 angegeben ist. Sie können zudem nach prinzipiell bekannten Verfahren hergestellt werden (siehe z.B. J. Org. Chem. Vol. 42, No. 9,1977).

Die Amine der Formel (II) können auf konventionelle Weise zu den entsprechenden Verbindungen der Formel (II)" acetyliert bzw. formyliert werden.

Die Verbindungen der Formel (III) sind bekannt und können z.B. nach dem in der DE-A 27 37 738 angegebenen Verfahren hergestellt werden.

Bei der erfindungsgemäßen Umsetzung der substituierten Enamine mit einem Chlorierungsmittel arbeitet man bei Temperaturen zwischen -10°C und 0°C. Als Verdünnungsmittel bei der Umsetzung werden bevorzugt halogenierte aromatische oder aliphatische Kohlenwasserstoffe eingesetzt. Methylenchlorid und Chlorbenzol sind als Verdünnungsmittel besonders bevorzugt.

Bei der erfindungsgemäßen Umsetzung der substituierten Enamine mit einem Chlorierungsmittel betragen die Reaktionszeiten im Allgemeinen zwischen 1 Minute und 6 Stunden, vorzugsweise zwischen 2 und 120 Minuten. Das Edukt wird im Allgemeinen bei einer Konzentration von 11 bis 50 Gew. %, bevorzugt 20 - 30 Gew. % in dem Verdünnungsmittel vorgelegt.

Zur Durchführung des erfindungsgemäßen Verfahrens setzt man zur Herstellung der Verbindungen der Formel (I) bevorzugt das Reagenz im Überschuss ein. Im Allgemeinen werden je Mol Enamin der Formel (II) zwischen 0,8 und 10 Mol, vorzugsweise zwischen 1,0 und 1,1 Mol die Chlorierungsmittel eingesetzt.

Als Chlorierungsmittel kann beim erfindungsgemäßen Verfahren (elementares, gasförmiges) Chlor oder Sulfurylchlorid (SO₂Cl₂) eingesetzt werden. Es können aber auch andere Verbindungen verwendet werden, welche leicht Chlor abgeben. Besonders bevorzugt ist die Verwendung von Sulfurylchlorid.

Das erfindungsgemäße Verfahren wird im Allgemeinen unter Normaldruck durchgeführt. Es ist jedoch auch möglich, das erfindungsgemäße Verfahren unter erhöhtem oder vermindertem Druck - im Allgemeinen zwischen 0,1 bar und 10 bar - durchzuführen.

Bei der Herstellung der Enamine der Formel (II) aus den Verbindungen der Formel (III) arbeitet man im Allgemeinen bei Temperaturen zwischen 20°C und 118°C, vorzugsweise zwischen 50°C und 118°C; besonders bevorzugt bei Rückflusstemperatur des betreffenden Verdünnungsmittels. Geeignete Verdünnungsmittel sind neutrale organische Lösungsmittel, wie zum Beispiel C₁-C₄-Alkohole, insbesondere Methanol, Ethanol, oder auch polare organische Lösungsmittel wie zum Beispiel Aceton oder Acetonitril. Als Verdünnungsmittel können weiterhin in Mischungen mit den vorgenannten Verdünnungsmitteln auch Verdünnungsmittel eingesetzt werden, die bei der nachfolgenden Umsetzung mit den Chlorierungsmitteln verwendet werden. Besonders bevorzugt ist die Verwendung von Methanol und Ethanol.

Bei der Umsetzung der Verbindungen der Formel (III) zu den Enaminen der Formel (II) betragen die Reaktionszeiten im Allgemeinen zwischen 30 Minuten und 12 Stunden, vorzugsweise zwischen 20 und 240 Minuten.

Zur Durchführung des Verfahrens zur Herstellung der Verbindungen der Formel (II) setzt man bevorzugt das Reagenz im Überschuss ein. Im Allgemeinen werden je Mol der Verbindung der Formel (III) zwischen 0,8 und 10 Mol, vorzugsweise zwischen 1,2 und 3,0 Mol des Reagenzes, z.B. Ammoniumformiat oder -acetat, eingesetzt.

Alternativ kann das Ammoniumformiat oder -acetat auch in katalytischen Mengen eingesetzt werden, wenn man während der Reaktionszeit NH₃ zudosiert. Pro Mol Edukt werden dann bevorzugt 0,1 - 0,8 Mol des betreffenden Ammoniumsalzes (oder einer Mischung aus beiden) und 1 bis 3 Mol NH₃ eingesetzt.

Wenn als Reagenz dieser Reaktion nicht Ammoniumformiat oder- acetat direkt eingesetzt wird, sondern ein Gemisch aus Essigsäure und NH₃ bzw. Ameisensäure und NH₃ so werden pro mol Edukt bevorzugt 0,1 bis 1 Mol Säure und 1 bis 3 Mol NH₃ eingesetzt.

Das Verfahren zur Herstellung der Verbindungen der Formel (II) aus denen der Formel (III) wird im Allgemeinen unter Normaldruck durchgeführt. Es ist jedoch auch möglich, das Verfahren unter erhöhtem oder vermindertem Druck - im Allgemeinen zwischen 0,1 bar und 10 bar - durchzuführen.

Die aus den Verbindungen der Formel (III) hergestellten Enamine der Formel (II) können vor ihrem Einsatz in dem erfindungsgemäßen Verfahrensschritt isoliert werden. Es ist aber auch möglich die erhaltenen Verbindungen unmittelbar ohne Zwischenisolierung nach teilweisem, bevorzugt aber vollständigem Tausch des Verdünnungsmittel weiter mit dem Chlorierungsmittel umzusetzen.

Gegenstand der vorliegenden Erfindung ist auch eine Kombination des erfindungsgemäßen Verfahrensschritt mit dem oben angegebenen Verfahrensschritt zur Herstellung der Ausgangsverbindungen der Formel (II). Erfindungsgemäß ist auch der Verfahrensschritt zur Herstellung der Ausgangsverbindungen der Formel (II) allein, der besonders gut geeignet ist, die Verbindungen der Formel (II) in hohen Ausbeuten zu erhalten.

Es wurde demnach gefunden, dass man 4-Alkoxycarbonyl-3-aminothiophene der allgemeinen Formel (I) 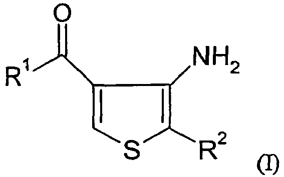 und/oder deren Hydrochloride der Formel (I)' 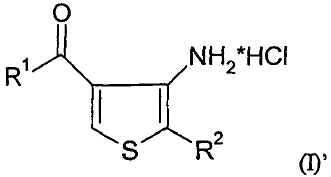 und/oder deren mono- oder bis-acetylierte bzw. mono- oder bis-formylierte Form der Formel (I)" 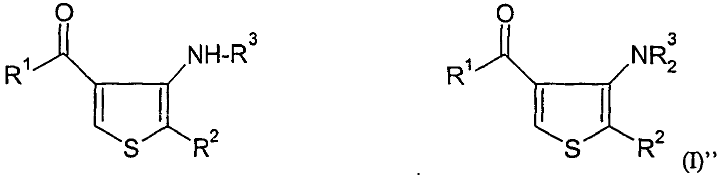 in welchen
- R¹: für gegebenenfalls durch Halogen oder C₁-C₄-Alkoxy substituiertes Alkoxy mit 1 bis 6 Kohlenstoffatomen steht,
- R²: für gegebenenfalls durch Halogen oder C₁-C₄-Alkoxy substituiertes Alkyl mit 1 bis 6 Kohlenstoffatomen oder für jeweils gegebenenfalls durch Halogen, C₁-C₄-Alkyl oder C₁-C₄-Alkoxy substituiertes Aryl oder Arylalkyl mit jeweils 6 oder 10 Kohlenstoffatomen in der Arylgruppe und gegebenenfalls 1 bis 4 Kohlenstoffatomen im Alkylteil steht,
- R³: für Acyl oder Formyl steht,
in sehr guten Ausbeuten und in hoher Reinheit erhält,
wenn man Verbindungen der Formel (III) 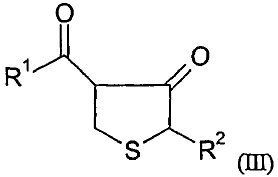 worin R¹ und R² die oben angegebene Bedeutung haben,
mit Ammoniumformiat gegebenenfalls in Gegenwart von NH₃, mit Ammoniumacetat gegebenenfalls in Gegenwart von NH₃, mit einem Gemisch aus Ameisensäure und NH₃ und/oder mit einem Gemisch aus Essigsäuresäure und NH₃ in Gegenwart eines oder mehrerer Verdünnungsmittel umsetzt,
und die erhaltenen Zwischenverbindungen der Formel (II) 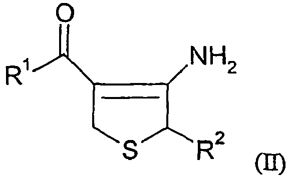 in welcher R¹ und R² die oben angegebene Bedeutung haben,
direkt oder nach nachfolgender Acetylierung bzw. Formylierung zu einer Verbindung der Formel (II)"
mit einem Chlorierungsmittel in Gegenwart eines oder mehrerer Verdünnungsmittel umsetzt.

### Herstellungsbeispiele:

### Beispiel 1

### Herstellung des Vorprodukts der Formel (II)

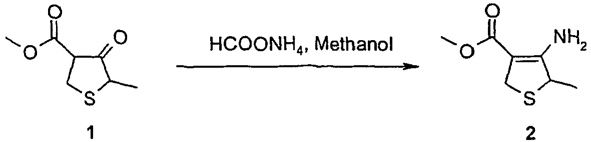

38,1 g 1,4-Anhydro-2,5-dideoxy-2-(methoxycarbonyl)-1-thiopent-3-ulose (1, 0,2 mol, Gehalt: 91,5 %) und 37,9 g Ammoniumformiat (0,6 mol) wurden in 330 ml Methanol vorgelegt und anschließend für 15 h auf Rückflußtemperatur erwärmt. Das Lösungsmittel wurde unter vermindertem Druck abdestilliert und der Rückstand in 200 ml Wasser aufgenommen. Nach einer Extraktion mit zweimal 200 ml CH₂Cl₂, Trocknung der vereinigten organischen Phasen (Na₂SO₄) und Abdestillieren des Lösungsmittels wurden 37 g Methyl-4-amino-5-methyl-2,5-dihydrothiophene-3-carboxylat (2) erhalten (97 % d. Th., Gehalt: 91 %, Fp.: 60 °C, ¹H NMR (400 MHz, d³-CD₃CN): 1,46 (d, 3H, *J*= 6,8 Hz), 3,63 (dd, 1H, *J₁* = 12 Hz, *J₂* = 0,9 Hz), 3,64 (s, 3H), 3,76 (dd, 1H, *J₁* = 12 Hz, *J₂* = 3,5 Hz), 4,11 - 4,19 (m, 1H)).

### Beispiel 2

### Herstellung des Vorprodukts der Formel (II)

20 g 1,4-Anhydro-2,5-dideoxy-2-(methoxycarbonyl)-1-thiopent-3-ulose (1, 87 mmol, Gehalt: 72,6 %) und 3,6 g Ammoniumacetat (44 mmol) wurden in 55 ml Methanol vorgelegt. Die Reaktionsmischung wurde für 2 h auf Rückfluß erwärmt. Anschließend wurden bei Rückflußtemperatur über einen Zeitraum von 30 min 1,8 g Ammoniakgas (105 mmol) eingeleitet. Die Reaktionsmischung wurde dann für weitere 7 h unter Rückfluß gerührt. Zur Aufarbeitung wurde analog dem Beispiel 1 verfahren. Man erhielt 19,3 g Methyl-4-amino-5-methyl-2,5-dihydrothiophene-3-carboxylat (2) (96 % d. Th., Gehalt: 73,6 %).

### Beispiel 3

### Herstellung des Vorprodukts der Formel (II)

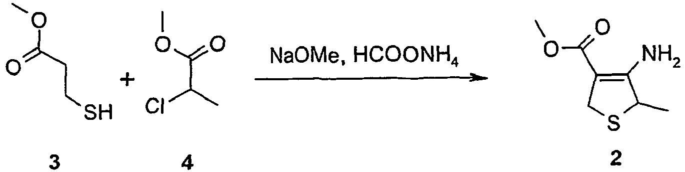

Bei 0 - 10 °C wurden 61,3 g 3-Mercaptopropionsäuremethylester (3, 0,5 mol, Gehalt: 98 %) über 35 min in 90 g einer 30 %igen Lösung von NaOMe in Methanol (0,5 mol) zugetropft. Nach der Zugabe wurde für 10 min bei 0 - 10°C nachgerührt und anschließend bei 0 - 5 °C 63,2 g 2-Chlorpropionsäuremethylester (**4**, 0,5 mol, Gehalt: 97 %) über 50 min zudosiert. Die Reaktionsmischung wurde 30 min bei 0 - 5 °C nachgerührt, bevor 500 ml Xylol zugesetzt wurden. Anschließend wurde das Methanol weitgehend abdestilliert. In die erhaltene Suspension wurden über 75 min bei ca. 90 °C 99 g einer 30 %igen Lösung von NaOMe in Methanol (0,55 mol) zugetropft und gleichzeitig weiter Methanol abdestilliert. Die Reaktionsmischung wurde dann unter Argon auf 80 °C abgekühlt bevor 33 g Essigsäure (0,55 mol) zudosiert wurden. Nach beendeter Zugabe wurden bei 70 °C 500 ml Wasser zugesetzt und anschließend auf Raumtemperatur abgekühlt. Nach der Trennung der Phasen wurde die wässrige Phase noch einmal mit 250 ml Xylol nachextrahiert und anschließend die vereinigten org. Phasen nochmals mit 100 ml Wasser gewaschen. Die org. Phase wurde getrocknet (Na₂SO₄) und unter vermindertem Druck eingeengt. Der Rückstand wurde mit 830 ml Methanol und 94,6 g Ammoniumformiat (1,5 mol) versetzt und dann auf Rückfluß erwärmt. Die Aufarbeitung erfolgte nach 12 h unter Rückfluß durch weitgehendes Einengen. Der Rückstand wurde in 500 ml Wasser aufgenommen und dreimal mit je 200 ml Dichlormethan extrahiert. Die vereinigten org. Phasen wurden getrocknet (Na₂SO₄). Nach Abdestillieren des Lösungsmittels unter vermindertem Druck erhielt man 81 g Methyl-4-amino-5-methyl-2,5-dihydrothiophene-3-carboxylat (**2**) (68 % d. Th. über alle Stufen, Gehalt: 72,5 %).

### Beispiel 4

### Herstellung des Vorprodukts der Formel (II)

Analog Beispiel 3 kann die Reaktion auch mit Chlorbenzol an Stelle von Xylol geführt werden. Die Ausbeute ist mit der Reaktionsführung in Xylol identisch.

### Beispiel 5

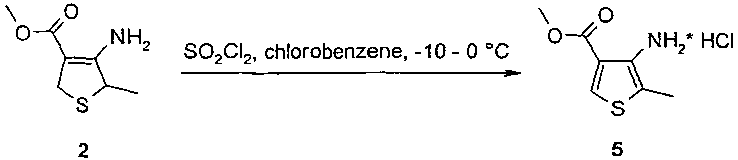

80,7 g Methyl 4-amino-5-methyl-2,5-dihydrothiophene-3-carboxylat (**2**) (338 mmol, Gehalt: 72,6 %) wurden in 88 ml Chlorbenzol gelöst und auf -10 °C gekühlt. Zu dieser Lösung wurde über einen Zeitraum von 1 h eine Lösung von 50,2 g Sulfurylchlorid (372 mmol) in 59 ml Chlorbenzol im Temperaturbereich von -10 - 0 °C zugetropft. Die Reaktionsmischung wurde anschließend noch für 1 h bei -10 - 0 °C gerührt und dann auf Raumtemperatur erwärmt. Zur Aufarbeitung wurde die Reaktionsmischung unter vermindertem Druck eingeengt. Man erhielt 95 g Methyl 4-amino-5-methylthiophene-3-carboxylat Hydrochlorid (**5**) (96 % d. Th., Gehalt: 58,7 %, bestimmt als freies Amin). Eine Charakterisierung des Produkts erfolgte nach Freisetzung des Amins mit NaHCO₃-Lösung (Fp.: 65 °C; ¹H NMR (400 MHz, d³-DMSO): 2,62 (s), 4,25 (s), 5,18 (bs), 8,22 (s)).

Aminhydrochlorid: ¹H NMR (400 MHz, d⁶-DMSO): 2,44 (s), 3,83 (s), 8,21 (s)).

### Beispiel 6

11,5 g Methyl 4-amino-5-methyl-2,5-dihydrothiophene-3-carboxylat (**2**) (60 mmol, Gehalt: 90 %) wurden in 25 ml CH₂Cl₂ gelöst und auf -10°C gekühlt. Zu dieser Lösung wurde über einen Zeitraum von 2 h eine Lösung von 8,9 g Sulfurylchlorid (66 mmol) in 10 ml CH₂Cl₂ im Temperaturbereich von -10 - 0°C zugetropft. Die Reaktionsmischung wurde anschließend noch für 1 h bei -10 - 0 °C gerührt und dann auf Raumtemperatur erwärmt. Zur Aufarbeitung wurde die Reaktionmischung unter vermindertem Druck eingeengt. Man erhielt 14,7 g Methyl 4-amino-5-methylthiophene-3-carboxylat Hydrochlorid (**5**) (89 % d. Th., Gehalt: 62,5 %, bestimmt als freies Amin).

### Beispiel 7

5,6 g Methyl 4-amino-5-methyl-2,5-dihydrothiophene-3-carboxylat (2) (30 mmol, Gehalt: 93 %) wurden in 30 ml CH₂Cl₂ gelöst. In diese Lösung wurden bei Raumtemperatur über einen Zeitraum von 60 min 2,3 g Chlor (33 mmol) eingeleitet. Die Reaktionsmischung wurde anschließend noch für 12 h gerührt und dann zur Aufarbeitung unter vermindertem Druck eingeengt. Man erhielt 7,2 g Methyl 4-amino-5-methylthiophene-3-carboxylat-hydrochlorid (**5**) (59 % d. Th., Gehalt: 42 %, bestimmt als freies Amin).

## Patentansprüche

1. Verfahren zum Herstellen von 4-Alkoxycarbonyl-3-aminothiophenen der allgemeinen Formel (I) 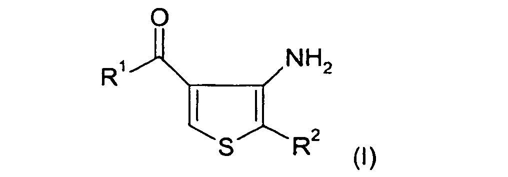 und/oder deren Hydrochloride der Formel (I)' 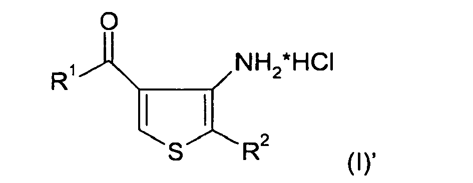 und/oder deren mono- oder bis-acetylierte bzw.mono- oder bis-formylierte Form der Formeln (I)" 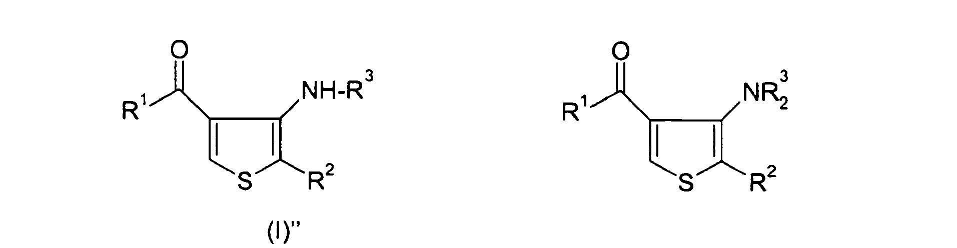 in welchen
R¹ für gegebenenfalls durch Halogen oder C₁-C₄-Alkoxy substituiertes Alkoxy mit 1 bis 6 Kohlenstoffatomen steht,
R² für gegebenenfalls durch Halogen oder C₁-C₄-Alkoxy substituiertes Alkyl mit 1 bis 6 Kohlenstoffatomen oder für jeweils gegebenenfalls durch Halogen, C₁-C₄-Alkyl oder C₁-C₄-Alkoxy substituiertes Aryl oder Arylalkylmit jeweils 6 oder 10 Kohlenstoffatomen in der Arylgruppe und gegebenenfalls 1 bis 4 Kohlenstoffatomen im Alkylteil steht, und R³ für einen Acyl- oder Formyl-Substituent steht, **dadurch gekennzeichnet, dass** Enamine der Formel (II) 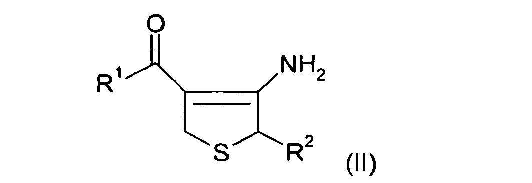
und/oder deren deren mono- oder bis-acetylierte bzw. mono- oder bis-formylierte Form der Formel (II)" 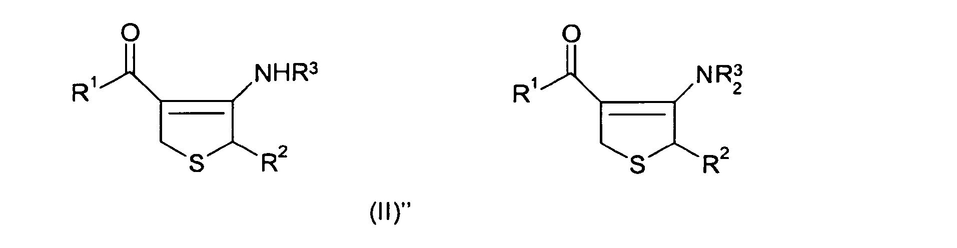 in welchen R¹, R² und R³ jeweils die zuvor angegebene Bedeutung haben,
mit einem Chlorierungsmittel in Gegenwart eines oder mehrerer Verdünnungsmittel ohne Hilfsbasenzusatz bei Temperaturen zwischen -10°C und 0°C umgesetzt werden.

2. Verfahren nach Anspruch 1, **dadurch gekennzeichnet, dass** in der Formel (I)
R¹ für C₁-C₄-Alkoxy,
R² für C₁-C₄-Alkyl und
R³ für Acyl- oder Formyl steht.

3. Verfahren nach Anspruch 1 oder 2, **dadurch gekennzeichnet, dass** das Verdünnungsmittel aus den Lösungsmitteln Chlorbenzol oder Methylenchlorid ausgewählt ist.

4. Verfahren gemäß einem der Ansprüche 1 bis 3, **dadurch gekennzeichnet, dass** das Enamin der Formel (II) direkt oder als Vorstufe für seine acetylierte Form der Formel (II)", in einem vorhergehenden Verfahrenschritt aus einer Verbindung der Formel (III) 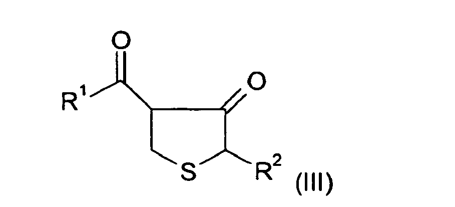 worin R¹ und R² die in Anspruch 1 angegebene Bedeutung haben,
durch Umsetzung mit Ammoniumformiat gegebenenfalls in Gegenwart von NH₃, mit Ammoniumacetat gegebenenfalls in Gegenwart von NH₃, mit einem Gemisch aus Ameisensäure und NH₃ und/oder mit einem Gemisch aus Essigsäuresäure und NH₃ in Gegenwart eines oder mehrerer Verdünnungsmittel erhalten wurde.

5. Verfahren nach Anspruch 4, **dadurch gekennzeichnet, dass** das in dem vorgehenden Verfahrensschritt hergestellte Enamin nicht isoliert wird.

6. Verfahren nach Anspruch 1, **dadurch gekennzeichnet, dass** das Chlorierungsmittel Sulfurylchlorid ist.

7. Verfahren nach Anspruch 4, **dadurch gekennzeichnet, dass** die Verbindungen der Formel (III) mit NH₃ in Gegenwart einer katalytischen Menge Ammoniumformiat oder Ammoniumacetat umgesetzt werden.

8. Verfahren nach Anspruch 7, **dadurch gekennzeichnet, dass** 0,1 - 0,8 Mol des Ammoniumsalzes als katalytische Menge verwendet werden.

## Claims

1. A process for preparing 4-alkoxycarbonyl-3-aminothiophenes of the general formula (I) 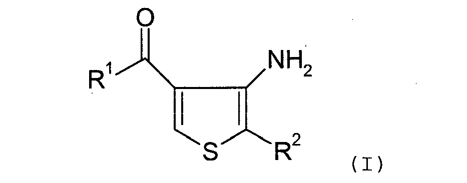 and/or their hydrochlorides of the formula (I)' 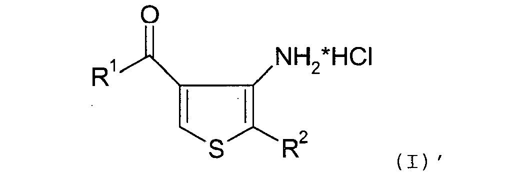 and/or their mono- or bisacetylated or mono- or bisformylated form of the formulae (I)'' 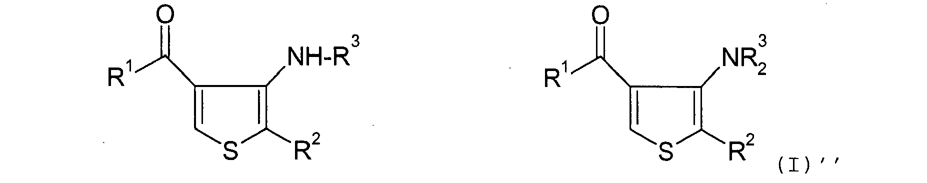 in which
R¹ is optionally halogen- or C₁-C₄-alkoxy-substituted
alkoxy having from 1 to 6 carbon atoms,
R² is optionally halogen- or C₁-C₄-alkoxy-substituted
alkyl having from 1 to 6 carbon atoms or in each case optionally halogen-, C₁-C₄-alkyl- or C₁-C₄-alkoxy-substituted aryl or arylalkyl having in each case 6 or 10 carbon atoms in the aryl group and, if appropriate, from 1 to 4 carbon atoms in the alkyl moiety, and
R³ is an acyl or formyl substituent,
which comprises
reacting enamines of the formula (II) 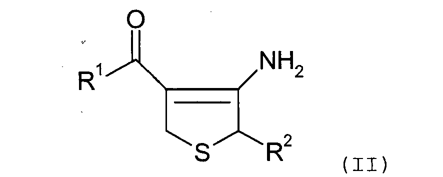 and/or their mono- or bisacetylated or mono- or bisformylated form of the formula (II)'' 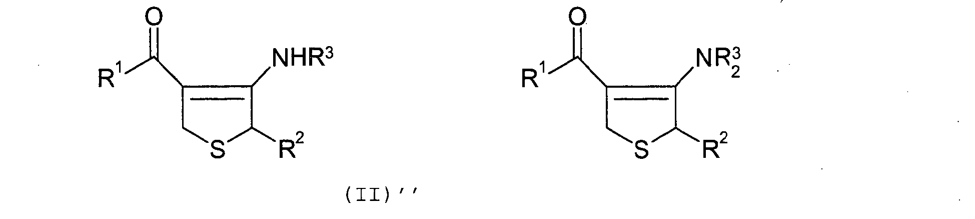 in which R¹, R² and R³ are each as defined above
with a chlorinating agent in the presence of one or more diluents without addition of an auxiliary base at temperatures between -10°C and 0°C.

2. The process as claimed in claim 1, wherein, in the formula (I),
R¹ is C₁-C₄-alkoxy,
R² is C₁-C₄-alkyl and
R³ is acyl or formyl.

3. The process as claimed in claim 1 or 2, wherein the diluent is selected from the solvents chlorobenzene or methylene chloride.

4. The process as claimed in one of claims 1 to 3, wherein the enamine of the formula (II) has been obtained directly or as a precursor for its acetylated form of the formula (II)" in a preceding process step from a compound of the formula (III) 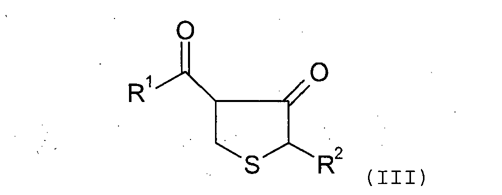 in which R¹ and R² are each as defined in claim 1 by reacting with ammonium formate, optionally in the presence of NH₃, with ammonium acetate, optionally in the presence of NH₃, with a mixture of formic acid and NH₃ and/or with a mixture of acetic acid and NH₃ in the presence of one or more diluents.

5. The process as claimed in claim 4, wherein the enamine prepared in the preceding process step is not isolated.

6. The process as claimed in claim 1, wherein the chlorinating agent is sulfuryl chloride.

7. The process as claimed in claim 4, wherein the compounds of the formula (III) are reacted with NH₃ in the presence of a catalytic amount of ammonium formate or ammonium acetate.

8. The process as claimed in claim 7, wherein 0.1-0.8 mol of the ammonium salt is used as a catalytic amount.

## Revendications

1. Procédé de fabrication de 4-alcoxycarbonyl-3-aminothiophènes de formule générale (I) 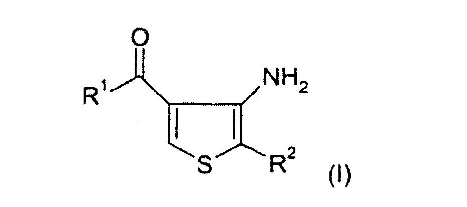 et/ou leurs chlorhydrates de formule (I)' 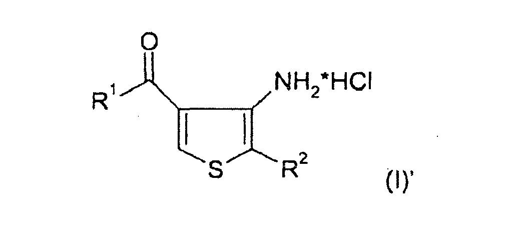 et/ou leur forme mono- ou bis-acétylée ou mono- ou bis-formylée de formule (I)'' 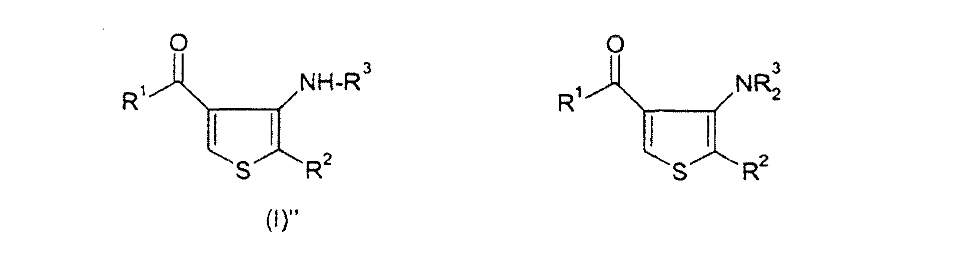 dans lesquelles R¹ représente alcoxy de 1 à 6 atomes de carbone éventuellement substitué par halogène ou alcoxy en C₁-C₄,
R² représente alkyle de 1 à 6 atomes de carbone éventuellement substitué par halogène ou alcoxy en C₁-C₄, ou aryle ou arylalkyle contenant chacun 6 ou 10 atomes de carbone dans le groupe aryle et éventuellement 1 à 4 atomes de carbone dans la partie alkyle, éventuellement substitué par halogène, alkyle en C₁-C₄ ou alcoxy en C₁-C₄, et
R³ représente un substituant acyle ou formyle, **caractérisé en ce que**
des énamines de formule (II) 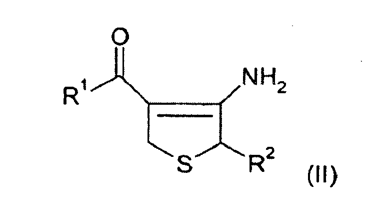 et/ou leur forme mono- ou bis-acétylée ou mono- ou bis-formylée de formule (II)'' 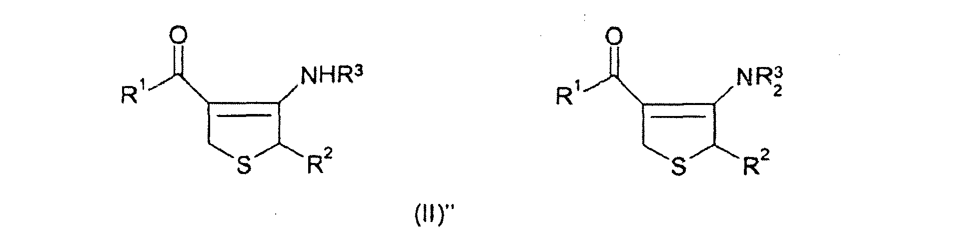 dans lesquelles R¹, R² et R³ ont chacun la signification donnée précédemment,
sont mises en réaction avec un agent de chloration en présence d'un ou de plusieurs diluants sans ajout de base auxiliaire à des températures comprises entre -10°C et 0°C.

2. Procédé selon la revendication 1, **caractérisé en ce que**, dans la formule (I)
R¹ représente alcoxy en C₁-C₄,
R² représente alkyle en C₁-C₄ et
R³ représente acyle ou formyle.

3. Procédé selon la revendication 1 ou 2, **caractérisé en ce que** le diluant est choisi parmi les solvants chlorobenzène ou chlorure de méthylène.

4. Procédé selon l'une quelconque des revendications 1 à 3, **caractérisé en ce que** l'énamine de formule (II) a été obtenue directement ou en tant que précurseur de sa forme acétylée de formule (II)'' par une étape de procédé antérieure à partir d'un composé de formule (III) 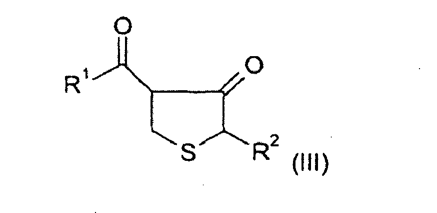 dans laquelle R¹ et R² ont la signification donnée dans la revendication 1,
par réaction avec du formiate d'ammonium éventuellement en présence de NH₃, avec de l'acétate d'ammonium éventuellement en présence de NH₃, avec un mélange d'acide formique et de NH₃ et/ou avec un mélange d'acide acétique et de NH₃ en présence d'un ou de plusieurs diluants.

5. Procédé selon la revendication 4, **caractérisé en ce que** l'énamine fabriquée lors de l'étape de procédé antérieure n'est pas isolée.

6. Procédé selon la revendication 1, **caractérisé en ce que** l'agent de chloration est le chlorure de sulfuryle.

7. Procédé selon la revendication 4, **caractérisé en ce que** les composés de formule (III) sont mis en réaction avec NH₃ en présence d'une quantité catalytique de formiate d'ammonium ou d'acétate d'ammonium.

8. Procédé selon la revendication 7, **caractérisé en ce que** 0,1 à 0,8 mol du sel d'ammonium est utilisé en tant que quantité catalytique.
